(19) Europäisches Patentamt
European Patent Office
Office européen des brevets

(11) Veröffentlichungsnummer: **0 291 807 B1**

(12) **EUROPÄISCHE PATENTSCHRIFT**

(45) Veröffentlichungstag der Patentschrift: **15.01.92**

(51) Int. Cl.[5]: **C07C 45/60**, C07C 47/198, C07C 47/277, C07D 335/02

(21) Anmeldenummer: **88107404.1**

(22) Anmeldetag: **07.05.88**

# (54) Umwandlung von 1,3-Dioxanen zu 4-Oxaaldehyden.

(30) Priorität: **12.05.87 DE 3715752**

(43) Veröffentlichungstag der Anmeldung: **23.11.88 Patentblatt 88/47**

(45) Bekanntmachung des Hinweises auf die Patenterteilung: **15.01.92 Patentblatt 92/03**

(84) Benannte Vertragsstaaten: **BE DE FR GB NL**

(56) Entgegenhaltungen:
**EP-A- 0 199 210**
**DE-A- 2 922 698**
**US-A- 3 676 500**

**J.A.C.S., Band 84, 5. September 1962, Seiten 3307-3319; C.S. RONDESTVEDT, Jr. et al.: "A new rearrangement. Catalytic isomerization of m-dioxanes to beta-alkoxy aldehydes. II. Scope and limitations"**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18c**
**W-6710 Frankenthal(DE)**
Erfinder: **Merger, Franz, Dr.**
**Max-Slevogt-Strasse 25**
**W-6710 Frankenthal(DE)**
Erfinder: **Lermer, Helmut, Dr.**

**W 3,4, 6800 Mannheim 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 291 807 B1

## Beschreibung

Die vorliegende Anmeldung betrifft ein Verfahren zur Herstellung von 4-Oxa-aldehyden durch katalytische Isomerisierung von 1 ,3-Dioxanen.

Es ist bekannt, daß 1,3-Dioxan und dessen Derivate zu β-Alkoxyaldehyden umgelagert werden können (J. Am. Chem. Soc. 892 (1960), 6419 und J. Am. - Chem. Soc. 84 (1962), 3307). Als Katalysatoren werden Silicagel und Bimsstein eingesetzt. Die verwendeten Katalysatoren zeigen starke Desaktivierungserscheinungen.Auch ihre Aktivität und Selektivität ist nicht befriedigend. Wegen der unzureichenden Spezifizierung des Naturproduktes Bimsstein, das je nach Herkunft unterschiedliche Zusammensetzungen hat, sind unkontrollierbare Einflüsse auf die Reaktion nicht zu vermeiden (Houben-Weyl, Methoden d. org. Chemie IV, 2, S. 149 (1955)).

In der DE-OS 29 22 698 wird ein Verfahren zur Herstellung von β-Alkoxypivalinaldehyd aus 1,3-Dioxanen unter Verwendung von Siliciumdioxid, dotiert mit Hydroxiden der Gruppe III A und/oder III B und Alkalihydroxid, als Katalysator beschrieben. Diese Katalysatoren, die nur einen graduellen Fortschritt bringen, unterscheiden sich von den vorher beschriebenen durch Neutralisation der aciden Zentren. Die für die Katalysatorfertigung notwendigen Verbindungen der reinen Lanthanide Praseodym und Neodym sind teure und nicht leicht zugängliche Chemikalien. Das bevorzugt benutzte handelsübliche Lanthaniden-Gemisch Didymium variiert in seiner Zusammensetzung, so daß die Reproduzierung der technische Katalysatoren schwierig ist. Die angegebenen Katalysatorstandzeiten liegen nur im Stundenbereich; über die Regenerierung der Katalysatoren wird keine Aussage gemacht.

Aus der EP-A-199 210 sind zeolithische Katalysatoren bekannt, die z.B. mit Phosphorsäure behandelt wurden. Hier wird die Phosphorsäure auf den bereits fertigen Zeolithen aufgebracht, z.B. durch Tränken dieser Zeolithe mit Phosphorsäure.

Der Erfindung liegt die Aufgabe zugrunde, ein Verfahren zu entwickeln, 4-Oxa-aldehyde aus den entsprechenden 1 ,3-Dioxanen herzustellen in Gegenwart von Katalysatoren, die sich durch einfache Verfügbarkeit, hohe Aktivität und leichte Regenerierbarkeit auszeichnen. Weiterhin sollen bei langen Katalysatorstandzeiten hohe Umsätze und Selektivitäten gewährleistet sein.

Gegenstand ist ein Verfahren zur Herstellung von 4-Oxa-aldehyden der Formel (I)

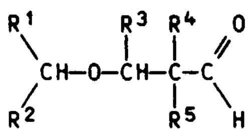

(I)

durch katalytische Isomerisierung von 1,3-Dioxanen, bei dem man 1,3-Dioxane der Formel (II)

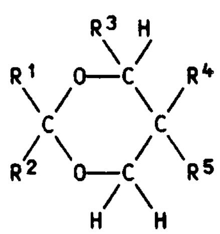

(II),

wobei die Reste $R^1$, $R^2$, $R^4$ und $R^5$ in den Formeln (I) und (II) untereinander gleich oder verschieden sind und für Wasserstoff, geradkettige oder verzweigte Alkyl-, Alkenyl oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl oder Alkenylarylreste mit 6 bis 16 Kohlenstoffatomen oder für heterocyclische Reste stehen und darüber hinaus jeweils die Reste $R^1$ und $R^2$ und/oder $R^4$ und $R^5$ zusammen mit dem C-Atom an das sie gebunden ist, ein Cycloalkan, Cycloalken oder einen Heterocyclus mit 5 bis 7 Ringgliedern bilden können, wobei die genannten Reste noch unter den Reaktionsbedingungen inerte Substituenten tragen können und wobei der Rest $R^3$ Wasserstoff oder einen geradkettigen oder verzweigten Alkylrest bedeutet, unter Verwendung von Phosphaten als Katalysatoren isomerisiert.

Bei dem erfindungsgemäßen Verfahren werden die eingangs gestellten Anforderungen an die Katalysatoren weitgehend erfüllt. Im Hinblick auf Lehren des Standes der Technik ist das Ergebnis, das bei dem Verfahren erzielt wird, besonders überraschend, weil diese Lehre gerade in entgegengesetzte Richtung, nämlich Ausschaltung der aciden Zentren, wies. Man konnte daher nicht erwarten, daß gerade mit sauren Phosphaten, die sich durch besonders hohe Acidität sowie im Falle der Phosphate mit Zeolithstruktur durch strenge Strukturparameter auszeichnen, in weiten Grenzen hervorragende Ergebnisse erhalten werden.

Die Umwandlung der 1,3-Dioxane zu 4-Oxa-aldehyden ist eine gute Methode, um beispielsweise die Ether der Hydroxyneoalkanale in hoher Selektivität bei hohem Umsatz herzustellen, die nach konventionellen Veretherungsmethoden nicht bzw. nur schwierig zugänglich sind.

Die Umwandlung läßt sich durch folgende Gleichung wiedergeben:

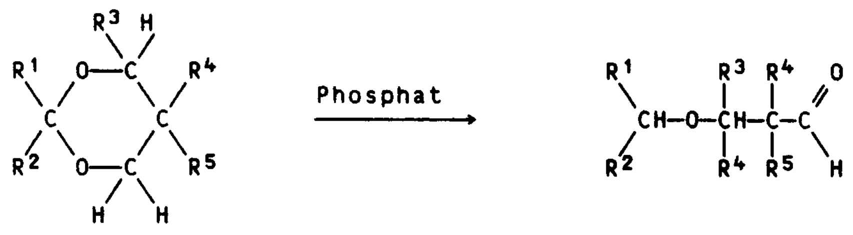


(II) (I)

Die als Ausgangsstoffe verwendeten 1,3-Dioxane der Formel (II) und dementsprechend die entstehenden 4-Oxa-aldehyde der Formel (I) enthalten die Reste $R^1$, $R^2$, $R^4$ und $R^5$, die gleich oder verschieden sind und unabhängig voneinander für Wasserstoff, verzweigte oder unverzweigte, Alkyl-, Alkenyl- oder Alkinylreste mit bis zu 18 Kohlenstoffatomen, insbesondere 1 bis 12, vorzugsweise 1 bis 6 Kohlenstoffatomen, Cycloalkyl-oder Cycloalkenylreste mit 5 bis 8, insbesondere 5 und 6 Kohlenstoffatome, Aryl-, Alkylaryl-, Aralkenyl- oder Alkenylarylreste mit 6 bis 16, insbesondere 6 bis 12 Kohlenstoffatomen, aromatische gesättigte und ungesättigte Heterocyclen, die ein oder mehrere Heteroatome, wie Stickstoff, Sauerstoff oder Schwefel enthalten. Die genannten Reste können noch unter den Reaktionsbedingungen inerte Substituenten tragen.

Die aus Verbindung (II) entstandenen Produkte (I) können ihrerseits mit einem Diol über ein Acetal der allgemeinen Formel (IV)

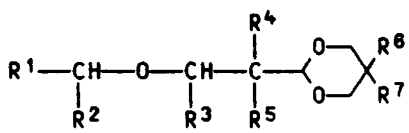

(IV),

in der die Reste $R^6$ und $R^7$ im oben angegebenen Definitionsbereich von $R^4$ und $R^5$ liegen, zu Aldehyden der Struktur

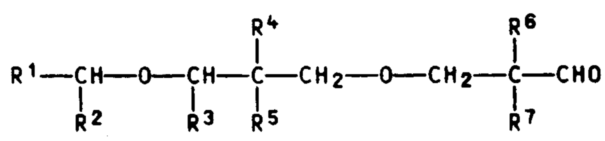

(III)

weiter aufgebaut werden.

Die Reste $R^1$ und $R^2$ und/oder $R^4$ und $R^5$ und/oder $R^6$ und $R^7$ können auch zusammen mit dem C-Atom, an das sie gebunden sind, ein Cycloalkan, Cycloalken oder einen Heterocyclus bilden. Als Rest $R^3$ kommen unabhängig von den übrigen Resten Wasserstoff sowie geradkettige oder verzweigte Alkylreste mit 1 bis 12, insbesondere 1 bis 8, vorzugsweise 1 bis 4 Kohlenstoffatomen in Betracht.

Alkyl-, Alkenyl- oder Alkinylreste sind z.B. Methyl-, Ethyl-, n-Propyl-, i-Propyl-, Propenyl-, i-Propenyl-, n-Butyl-, i-Butyl-, n-Butenyl-, i-Butenyl-, Pentyl-, Pentenyl-, Pentinyl-, Hexyl-, Hexenyl-, Heptyl-, Heptenyl-, Octyl-, Octenyl-, Nonyl-, Nonenyl-, Decyl-, Decenyl-, Dodecyl- oder Dodecenylreste. Die Alkyl-, Alkenyl- oder Alkinylreste können noch unter den Reaktionsbedingungen inerte Substituenten tragen, z.B. Halogen,

Alkoxy-oder Carboxygruppen.

Cycloalkylreste sind z.B. Cyclopentyl-, Cyclohexyl-, Cycloheptyl-, Cyclopentenyl- oder Cyclohexenylreste.

Als aromatische Reste kommen z.B. Phenyl-, Benzyl-, 2-Phenylethyl-, 3-Phenylpropyl-, 2-Phenylpropyl-, 4-Phenylbutyl-, 3-Phenylbutyl-, 2-Phenylbutyl- oder 3-Phenylbutenyl-Reste in Betracht, die gegebenenfalls noch durch unter den Reaktionsbedingungen inerte Reste substituiert sein können.

Heterocyclische bzw. heteroaromatische Reste sind z.B. Tetrahydrofuran-, Dihydrofuran-, Furan-, Tetrahydrothiophen(Thiophan)-, Dihydrothiophen-, Thiophen-, Pyridin-, Thiopyran. Diese Reste können noch durch unter den Reaktionsbedingungen inerte Reste, wie Alkylreste oder Halogenatome substituiert sein.

Für das erfindungsgemäße Verfahren besonders geeignete Ausgangsstoffe sind solche 1,3-Dioxane, in denen $R^3$ Wasserstoff bedeutet, während $R^4$ und $R^5$ einen der genannten organischen Reste darstellen.

Die Ausgangsverbindungen der Formel (II) lassen sich nach bekannten Methoden aus Aldehyden oder Ketonen oder deren leicht spaltbaren Derivaten, z.B. Dialkylketalen oder Acetalen mit 1,3-Diolen gemäß folgender Reaktionsgleichung herstellen.

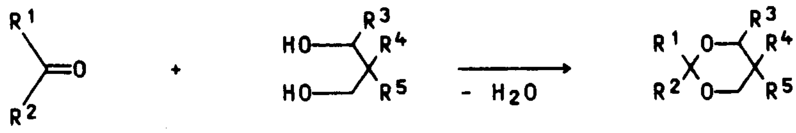


Als Diolkomponente kommen z.B. folgende Verbindungen in Betracht: Propandiol-1,3, 2-Methyl-, 2-Ethyl-, 2-Phenyl-, 2,2-Dimethyl-, 2,2-Diethyl-, 2-Methyl-2-ethyl-, 2-Methyl-2-propyl-, 2-Methyl-2-butyl-, 2-Methyl-2-phenyl und 2-Ethyl-2-butyl-propandiol-1,3, 1,1-Dimethylolcyclohexan und -pentan, 3,3-Dimethyloltetrahydrofuran und -pyran sowie 2,2,4-Trimethylpentadiol-1,3.

Geeignete Carbonylkomponenten sind z.B. aliphatische, aromatische oder heterocyclische Aldehyde und Ketone oder deren Acetale bzw. Ketale mit niedrig siedenden Alkoholen.

Gesättigte aliphatische Aldehyde sind z.B. Form-, Acet-, Propion- oder Butyraldehyd, Pentanal, Hexanal und höhere homologe n-Alkanale, wie Dekanal, Isobutyraldehyd, 2-Methylbutanal, 3-Methylbutanal, 3,3-Dimethylbutanal, 2-Methylpentanal, 2-Ethylhexanal und 2-Methyldekanal, Glyoxal, Methylglyoxal, Malondialdehyd, Succindialdehyd und Glutardialdehyd.

Heterocyclische Aldehyde sind z.B. Tetrahydrofuryl-2-aldehyd und -3-aldehyd, Tetrahydrothienyl-2- und -3-aldehyd, 5,6-Dihydropyranyl-6-aldehyd, 2,5-Dimethyl-5,6-dihydropyranyl-6-aldehyd, Furyl-2-aldehyd und -3-aldehyd. Thienyl-3-aldehyd, 2-, 3- oder 4-Pyridylaldedhyd.

Als Ketone kommen beispielsweise die folgenden Verbindungen in Betracht: Aceton, Methylethylketon, Diethylketon, Methylisopropylketon, Diisopropyl-und Diisobutylketon, Methylisobutylketon, Methoxyaceton, Methylvinylketon, Methylisopropenyl- und Methylisobutenylketon, Cyclopentanon, Cyclohexanon, Methylcyclopentanone, Methylcyclohexanone, Cyclohexenon, 3,5,5-Trimethylcyclohexenon-2, Methyl-, Ethyl- und Vinylphenylketon, Methylfurylketon, Acetylaceton und Acetessigester.

Weitere substituierte Alkanale sind beispielsweise 3-Hydroxy-2,2-dimethylpropanal, Methoxy- und Butoxypivalinaldehyd, 4-Acetoxybutyraldehyd und 5-Formylvaleriansäureethylester.

Weiterhin können ungesättigte Aldehyde verwendet werden, z.B. Acrolein, α-Methylacrolein, α-Ethylacrolein und höhere α-Alkyl-, Isoalkyl und Alkenylacroleine, wie But-2-enal, 2-Methyl-but-2-enal, 2-Methylpent-2-enal, 2-Ethyl-hex-2-enal, 2,2-Dimethyl-pent-4-enal, 2-Methyl-4-acetoxy-but-2-enal, 2-Methoxymethylacrolein, 2-(3-Methoxycarbonyl-propyl-)-acrolein oder 2-Methyl-4-chlor-but-2-enal.

Aromatische Aldehyde sind z.B. Benzaldehyd, p-Methoxybenzaldehyd, Phenylacetaldehyd, 2-Phenyl- und 3-Phenylpropanal, 2-Hydroxybenzaldehyd, 3-Hydroxy-4-methoxybenzaldehyd, Zimtaldehyd oder Benzylacrolein.

Als Katalysatoren für die erfindungsgemäße Umwandlung von 1,3-Dioxanen werden Phosphate verwendet, insbesondere geeignet sind Aluminiumphosphate, Siliciumaluminiumphosphate, Siliciumeisenaluminiumphosphate, Cerphosphate, Zirkonphosphate, Borphosphate, Eisenphosphate, Strontiumphosphate oder deren Gemische.

Als Aluminiumphosphat-Katalysatoren werden für das erfindungsgemäße Verfahren vorteilhaft unter hydrothermalen Bedingungen synthetisierte Aluminiumphosphate eingesetzt. Diese Aluminiumphosphate besitzen Zeolithstruktur.

Die unter hydrothermalen Bedingungen hergestellten Aluminiumphosphate sind z.B. APO-5, APO-9, APO-11, APO-12, APO-14, APO-21, APO-25, APO-31 und APO-33.

Das $AlPO_4$-5 (APO-5) kann man beispielsweise synthetisieren, indem man Orthophosphorsäure mit

Pseudoboehmit in Wasser homogen mischt; zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird getrocknet bei 100 bis 160 °C und calciniert bei 450 bis 550 °C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200 °C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidin-Lösung bei 150 bis 200 °C unter autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Diese Siliciumaluminiumphosphate besitzen Zeolithstruktur und werden durch Kristallisation aus einer wäßrigen Mischung bei 100 bis 250 °C und autogenem Druck während 2 h bis 2 Wochen hergestellt, wobei die Reaktionsmischung aus einer Silicium-, Aluminium- und Phosphorkomponente in wäßrigen aminoorganischen Lösungen umgesetzt wird.

So erhält man SAPO-5 beispielsweise durch Mischen von $SiO_2$ - suspendiert in wäßriger Tetrapropylammoniumhydroxid-Lösung - mit einer wäßrigen Suspension aus Pseudoboehmit und Orthophosphorsäure und anschließende Umsetzung bei 150 bis 200 °C während 20 bis 200 h unter autogenem Druck in einem Rührautoklaven. Die Trocknung des abfiltrierten Pulvers erfolgt bei 110 bis 168 °C und die Calcination bei 450 bis 550 °C.

Als Siliciumaluminiumphosphate sind auch z.B. ZYT-5, ZYT-6, ZYT-7, ZYT-9, ZYT-11 und ZYT-12 geeignet.

Weiterhin kann man als Phosphatkatalysatoren bei dem Verfahren gefällte Aluminiumphosphate einsetzen. Beispielsweise behält man ein derartiges Aluminiumphosphat, indem man 92 g Diammoniumhydrogenphosphat in 700 ml Wasser löst und zu dieser Lösung eine Lösung von 268 g $Al(NO_3)_3 \times H_2O$ in 780 ml Wasser über 2 h tropft. Dabei wird der pH-Wert durch gleichzeitige Zugabe von 25%iger $NH_3$-Lösung bei pH 8 gehalten. Der entstandene Niederschlag wird 12 h nachgerührt, dann abgesaugt und ausgewaschen. Er wird bei 60 °C/16 h getrocknet.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 258 bis 650 °C, vorzugsweise 300 bis 500 °C, herstellen.

Wenn bei der erfindungsgemäßen Verwendung der Phosphat-Katalysatoren eine durch Koksabscheidung bedingte Desaktivierung eintritt, empfiehlt es sich, die Phosphat-Katalysatoren durch Abbrennen der Koksablagerung mit Luft oder mit einem Luft/$N_2$-Gemisch bei 300 bis 550 °C zu regenerieren.

Durch partielle Verkokung (pre-coke) ist es möglich, die Aktivität des Katalysators für ein Selektivitätsoptimum des gewünschten Reaktionsproduktes einzustellen.

Um eine hohe Selektivität, hohen Umsatz sowie lange Standzeiten zu erreichen, ist es vorteilhaft, die Phosphat-Katalysatoren zu modifizieren. Eine geeignete Modifizierung der Katalysatoren besteht z.B. darin, daß man das unverformte oder verformte Phosphat mit Metallsalzen durch einen Ionenaustausch oder durch Imprägnierung dotiert. Als Metalle werden Alkalimetalle, wie Li, Cs, K, Erdalkalimetalle, wie Mg, Ca, Sr, Metalle der 3., 4. und 5. Hauptgruppe, wie Al, Ga, Ge, Sn, Pb, Ni, Übergangsmetalle der 4. - 8. Nebengruppe, wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe, wie Cu, Ag, Zn, seltene Erdmetalle, wie La, Ce, Pr, Nd, Er, Yb und U, eingesetzt.

Die hier beschriebenen Katalysatoren können wahlweise als 2- bis 4-mm-Stränge oder als Tabletten mit 3 bis 5 mm Durchmesser oder als Pulver mit Teilchengröße von 0,1 bis 0,5 mm oder als Wirbelkontakt eingesetzt werden.

Die für die erfindungsgemäße Umwandlung in der Regel gewählten Reaktionsbedingungen sind in der Gasphase 200 bis 500 °C, vorzugsweise 230 bis 400 °C, und eine Belastung WHSV = 0,1 bis 20 $h^{-1}$, bevorzugt 0,5 bis 5 $h^{-1}$ (g 1,3-Dioxan/g Katalysator und Stunde). Die Reaktion kann im Festbett oder Wirbelbett durchgeführt werden. Auch die Umsetzung in flüssiger Phase, in Suspensions-, Riesel- oder Sumpffahrweise, bei Temperaturen von 50 bis 200 °C ist möglich. Im allgemeinen steigt der Umsatz mit steigender Temperatur stark an, während die Selektivität in einen bestimmten Temperaturbereich nur wenig zurückgeht.

Das Verfahren wird in der Regel bei Normaldruck oder je nach Flüchtigkeit der Ausgangsverbindung bei vermindertem oder erhöhtem Druck durchgeführt, wobei die Durchführung vorzugsweise kontinuierlich erfolgt.

Schwerflüchtige oder feste Ausgangsstoffe kann man in gelöster Form, z.B. in THF-, Toluol- oder Petrolether-Lösung einsetzen. Es ist auch eine Verdünnung des Ausgangsstoffes mit derartigen Lösungsmitteln oder mit Inertgasen wie $N_2$, Ar, $H_2O$-Dampf möglich.

Nach der Umsetzung werden die entstandenen 4-Oxa-aldehyde nach den dafür üblichen Techniken, z.B. durch Destillation aus dem Reaktionsgemisch, isoliert; nicht umgesetzte 1,3-Dioxane werden gegebenenfalls in die Umsetzung zurückgeführt.

Die nach dem erfindungsgemäßen Verfahren zugänglichen Verbindungen und eine Reihe ihrer Derivate sind als biologisch aktive Verbindungen, z.B. als Bakterizide, von Interesse und darüber hinaus wertvolle Zwischenprodukte. Man kann sie in einfacher Weise zu Aminen, Alkoholen und Säuren nach geläufigen Methoden, z.B. durch Oxidation mit Sauerstoff oder durch Reduktion, z.B. durch katalytische Hydrierung oder aminierende Hydrierung weiterverarbeiten. Bei der Verbindung mit der Formel

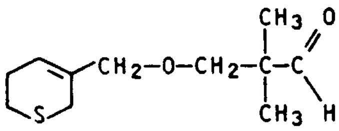

handelt es sich um eine neue Verbindung mit Smp. 53.8° C und Sdp. 121 bis 125° C.

Die folgenden Beispiele veranschaulichen die Erfindung. Das Vergleichsbeispiel 7 zeigt, daß die in der DE-OS 29 22 698 beschriebenen Katalysatoren nur bei kurzen Reaktionszeiten von 1 bis 2 Stunden wirksam sind, während nach 4 Stunden die Aktivität praktsich auf Null absinkt.

Beispiele 1 bis 18

Die Reaktionen werden unter isothermen Bedingungen in einem Rohrreaktor (Wendel, 0,6 cm Durchmesser, 90 cm Länge) in der Gasphase mindestens 6 Stunden lang durchgeführt. Die Trennung und Charakterisierung der Reaktionsprodukte erfolgte nach üblichen Methoden. Die quantitative Bestimmung der Reaktionsprodukte (I) und der Ausgangsstoffe (II) erfolgte gaschromatographisch.

Die in den Beispielen verwendeten Katalysatoren für die Umwandlung von 1,3-Dioxanen zu 4-Oxa-aldehyden sind:

Katalysator A

$AlPO_4$-5 (APO-5) wird synthetisiert, indem man 200 g 98%ige Phosphorsäure und 136 g Boehmit in 335 g Wasser löst bzw. suspendiert, hierzu 678 g einer 30%igen wäßrigen Tetrapropylammoniumhydroxid-Lösung zugibt und diese Mischung in einem Rührautoklaven bei 150° C in 43 h unter autogenem Druck umsetzt. Nach Abfiltrieren wird das kristalline Material bei 120° C getrocknet und 500° C/16 h calciniert. Das so synthetisierte $AlPO_4$-5 enthält 45,5 Gew.% $Al_2O_3$ und 46,5 Gew.% $P_2O_5$. Dieses Material wird im Pseudo-Boehmit im Massenverhältnis 60 : 40 zu 2-mm-Strängen verformt, abermals bei 120° C getrocknet und bei 500° C/16 h calciniert.

Katalysator B

Bei der Synthese von $AlPO_4$-21 (APO-21) erfolgt durch Zusammenrühren von 200 g 98%ige Phosphorsäure, 156 g gefälltes Aluminiumhydroxid und 71 g Pyrrolidin in 900 g Wasser und anschließende Reaktion bei 200° C unter autogenem Druck während 91 Std.. Das bei 120° C getrocknete und bei 500° C calcinierte Produkt enthält 56,6 Gew.% $P_2O_5$ und 43,4 Gew.% $Al_2O_3$. Dieser $AlPO_4$-21 wird mit Verstrangungshilfsmitteln zu 2-mm-Strängen verformt, bei 110° C getrocknet und bei 500° C/16 h calciniert.

Katalysator C

$AlPO_4$-9 (APO-9) wird synthetisiert, indem man 200 g 98%ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200° C während 336 Stunden unter autogenem Druck umsetzt. Nach Abfiltrieren wird das kristalline Material bei 120° C getrocknet und 500° C/16 h calciniert. Das so synthetisierte $AlPO_4$-9 enthält 49,0 Gew.% $P_2O_5$, 37,1 Gew.% $Al_2O_3$.

Dieses Material wird mit Verstrangungshilfsmitteln zu 3mm-Strängen verformt, abermals bei 120° C getrocknet und bei 500° C/6 h calciniert.

Katalysator D

SAPO-5 wird hergestellt aus einer Mischung aus 200 g 98%ige Phosphorsäure, 136 g Boehmit, 60 g Kieselsol (30%ig), 287 g Tripropylamin und 587 g $H_2O$. Diese Mischung wird bei 150 °C während 168 Stunden unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, bei 120 °C getrocknet und bei 500 °C calciniert.

Katalysator E

Im Handel erhältliches Zirkonphosphat $Zr_3(PO_4)_4$ in Reinsubstanz wird verformt.

Katalysator F

Katalysator E ist ein gefälltes Aluminiumphosphat, das durch Fällung aus $AL(NO_3)_3$-$H_3PO_4$-Lösung mit $NH_3$ bei pH = 6-7 erhalten wird. Nach Abfiltrieren des Niederschlags wird bei 110 °C getrocknet und bei 500 °C calciniert. Katalysator F enthält 28,5 Gew.% Al und 13,2 Gew.% P.

Katalysator G (Vergleichskatalysator nach DOS 29 22 698)

51 g eines handelsüblichen $SiO_2$ mit der in DOS 29 22 698 angegebenen Spezifikation (D11-11®) wird mit einer Lösung aus 51 g 0,1n $CH_3COOH$, 3,19 g Pr $(NO_3)_3$ x 5 $H_2O$, 3,21 g Nd $(NO_3)_3$ x 5 $H_2O$ und 2,66 g $CH_3COOK$ imprägniert, getrocknet und bei 600 °C/4 h calciniert.

Die mit diesen Katalysatoren erzielten Versuchsergebnisse sind in nachfolgenden Tabellen aufgelistet.

Tabelle 1

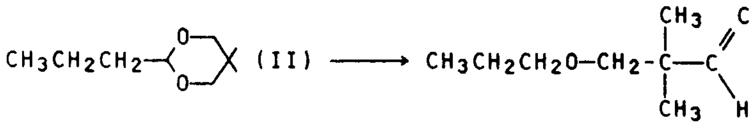

(II) → (I)

| Beispiel | 1 | 2 | 3 | 4 | 5 | 6 | 7 1) |
|---|---|---|---|---|---|---|---|
| Katalysator | A | B | D | E | E | F | G |
| Temperatur [°C] | 275 | 300 | 300 | 275 | 300 | 275 | 275 |
| WHSV [$h^{-1}$] | 2 | 3 | 3 | 2 | 2 | 2 | 2 |
| Umsatz [%] | | | | | | | |
| (II) | 48,9 | 49,6 | 62,7 | 67,5 | 83,4 | 84,1 | 23,0 |
| Selektivität [%] | | | | | | | |
| (I) | 72,2 | 66,7 | 80,5 | 82,7 | 76,9 | 53,7 | 11,5 |

1) Vergleichsbeispiel

Tabelle 1 (Fortsetzung)

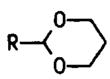 (II)

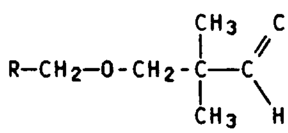 (I)

| Beispiel | 8 | 9 | 10 | 11 | 12 | 13 | 14 |
|---|---|---|---|---|---|---|---|
| R | 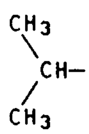 | | | 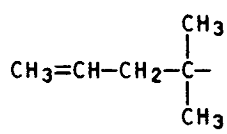 | | 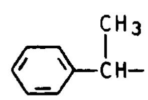 | |
| Katalysator | D | E | F | D | E | D | E |
| Temperatur [°C] | 275 | 300 | 275 | 275 | 300 | 275 | 300 |
| WHSV [$h^{-1}$] | 2 | 2 | 2 | 2 | 2 | 2 | 2 |
| Umsatz [%] | | | | | | | |
| (II) | 44,9 | 70,3 | 45,9 | 22,6 | 35,6 | 62,1 | 73,2 |
| Selektivität [%] | | | | | | | |
| (I) | 89,8 | 80,5 | 73,4 | 80,1 | 77,5 | 68,4 | 80,2 |

Tabelle 1 (Fortsetzung)

| Beispiel | 15 2) | 16 2) | 17 3) | 18 3) |
|---|---|---|---|---|
| R | 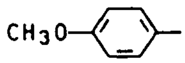 | | 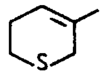 | → |
| Katalysator | E | C | D | F |
| Temperatur [°C] | 275 | 300 | 275 | 275 |
| WHSV [$h^{-1}$] | 2 | 2 | 3 | 2 |
| Umsatz [%] | | | | |
| (II) | 85,9 | 51,1 | 23,9 | 48,6 |
| Selektivität [%] | | | | |
| (I) | 51,9 | 71,6 | 77,8 | 68,2 |

2) Edukt verdünnt mit THF im Gewichtsverhältnis 25 : 75

3) Edukt verdünnt mit THF im Gewichtsverhältnis 50 : 50

## Patentansprüche

**1.** Verfahren zur Herstellung von 4-Oxa-aldehyden der Formel (I)

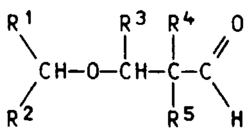

(I)

durch katalytische Isomerisierung von 1,3-Dioxanen, dadurch gekennzeichnet, daß man 1,3-Dioxane der Formel (II)

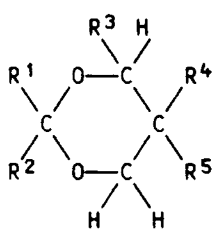

(II),

wobei die Reste $R^1$, $R^2$, $R^4$ und $R^5$ in den Formeln (I) und (II) untereinander gleich oder verschieden sind und für Wasserstoff, geradkettige oder verzweigte Alkyl-, Alkenyl- oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl- oder Alkenylarylreste mit 5 bis 16 Kohlenstoffatomen oder für heterocyclische Reste stehen und darüber hinaus jeweils die Reste $R^1$ und $R^2$ und/oder $R^4$ und $R^5$ zusammen mit dem C-Atom, an das sie gebunden sind, ein Cycloalkan, Cycloalken oder einen Heterocyclus bilden können, wobei die genannten Reste noch unter den Reaktionsbedingungen inerte Substituenten tragen können und wobei der Rest $R^3$ Wasserstoff oder einen geradkettigen oder verzweigten Alkylrest bedeutet, unter Verwendung von Phosphaten als Katalysatoren isomerisiert.

2. Verfahren nach Anspruch 1, dadurch gekennzeichnet, daß Acetale oder Ketale des Propandiols-1,3, des 2-Methyl-, 2,2-Dimethyl-, 2-Methyl-2-Ethyl-, 2-Methyl-2-propyl-, 2-Methyl-2-butyl-, 2-Methyl-2-phenyl-, 1-Isopropyl-2,2-dimethyl-propandiols-1,3 oder des 1,1-Dimethylolcyclohexans oder -pentans isomerisiert werden.

3. Verfahren nach Anspruch 1 und 2, dadurch gekennzeichnet, daß man als Katalysatoren Phosphate mit Zeolithstruktur verwendet.

4. Verfahren nach den Ansprüchen 1 - 3, dadurch gekennzeichnet, daß man als Katalysatoren hydrothermal hergestellte Aluminiumphosphate oder Siliciumaluminiumphosphate oder Siliciumeisenaluminiumphosphate oder Eisenaluminiumphosphate verwendet.

5. Verfahren nach Anspruch 1 und 2, dadurch gekennzeichnet, daß man als Katalysatoren Phosphate des Al, B, Fe, Zr, Sr, Ce oder deren Gemische verwendet.

6. Verfahren nach Anspruch 1 bis 5, dadurch gekennzeichnet, daß man die Isomerisierung in der Gasphase ausführt.

## Claims

1. A process for the preparation of a 4-oxaaldehyde of the formula (I)

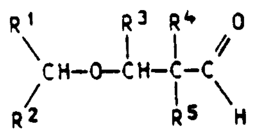

(I)

by catalytic isomerization of a 1,3-dioxane, wherein a 1,3-dioxane of the formula (II)

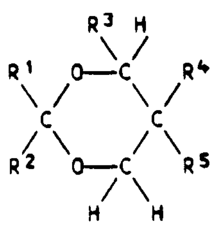

(II)

where $R^1$, $R^2$, $R^4$ and $R^5$ in the formulae (I) and (II) are identical or different and are each hydrogen, a straight-chain or branched alkyl, alkenyl or alkynyl radical of not more than 18 carbon atoms, a cycloalkyl or cycloalkenyl radical of 5 to 8 carbon atoms, an aryl, alkylaryl, aralkyl, aralkenyl or alkenylaryl radical of 5 to 16 carbon atoms or a heterocyclic radical and moreover $R^1$ and $R^2$ and/or $R^4$ and $R^5$ together with the carbon atom to which they are bonded may form a cycloalkane, cycloalkene or a heterocyclic structure, and the stated radicals may furthermore carry substituents which are inert under the reaction conditions and $R^3$ is hydrogen or a straight-chain or branched alkyl radical, is isomerized using a phosphate as a catalyst.

2. A process as claimed in claim 1, wherein an acetal or ketal of propane-1,3-diol, 2-methyl-, 2,2-dimethyl-, 2-methyl-, 2-ethyl-, 2-methyl-2-propyl-, 2-methyl-2-butyl-, 2-methyl-2-phenyl- or 1-isopropyl-2,2-dimethyl-propane-1,3-diol or of 1,1-dimethylolcyclohexane or -pentane is isomerized.

3. A process as claimed in claims 1 and 2, wherein the catalyst used is a phosphate having a zeolite structure.

4. A process as claimed in claims 1 to 3, wherein the catalyst used is a hydrothermally prepared aluminum phosphate or silicon aluminum phosphate or silicon iron aluminum phosphate or iron

aluminum phosphate.

5. A process as claimed in claims 1 and 2, wherein the catalyst used is a phosphate of Al, B, Fe, Zr, Sr or Ce or a mixture of these.

6. A process as claimed in claims 1 to 5, wherein the isomerization is carried out in the gas phase.

**Revendications**

1. Procédé de préparation de 4-oxa-aldéhydes de formule (I)

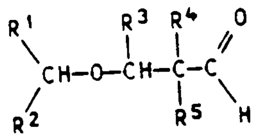

(I)

par isomérisation catalytique de 1,3-dioxanes, caractérisé en ce que l'on isomérise, avec emploi de phosphates comme catalyseurs, des 1,3-dioxanes de la formule (II)

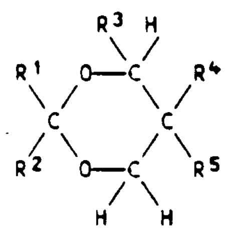

(II),

dans laquelle les restes $R^1$, $R^2$, $R^4$ et $R^5$ dans les formules (I) et (II) sont identiques entre eux ou différents et sont mis pour un atome d'hydrogène, des restes alkyle, alcényle ou alcinyle à chaîne droite ou ramifiée contenant jusqu'à 18 atomes de carbone, des restes cycloalkyle ou cycloalcényle contenant 5 à 8 atomes de carbone, des restes aryle, alkylaryle, aralkyle ou alcénylaryle contenant 5 à 16 atomes de carbone, ou pour des restes hétérocycliques, et en outre, suivant le cas, les restes $R^1$ et $R^2$ et/ou $R^4$ et $R^5$ peuvent former, conjointement avec l'atome de carbone auquel ils sont liés, un cycloalcane, un cycloalcène ou un hétérocycle, les restes précités pouvant encore porter des substituants inertes dans les conditions de la réaction, et le reste $R^3$ désignant de l'hydrogène ou un reste alkyle à chaîne droite ou ramifiée.

2. Procédé suivant la revendication 1, caractérise en ce que l'on isomérise des acétals ou des cétals du propanediol-1,3, du 2-méthyl-, 2,2-diméthyl-, 2-méthyl-2-éthyl-,2-méthyl-2-propyl-, 2-méthyl-2-butyl-, 2-méthyl-2-phényl-, 1-isopropyl-2,2-diméthyl-propanediol-1,3, ou du 1,1-diméthylol-cyclohexane ou -pentane.

3. Procédé suivant la revendication 1 ou 2, caractérisé en ce que l'on utilise, comme catalyseurs, des phosphates ayant une structure de zéolithe.

4. Procédé suivant les revendications 1 - 3, caractérisé en ce que l'on utilise, comme catalyseurs, des phosphates d'aluminium préparés par voie hydrothermale ou des phosphates de silicium et d'aluminium ou des phosphates de silicium, de fer et d'aluminium ou des phosphates de fer et d'aluminium.

5. Procédé suivant la revendication 1 ou 2, caractérisé en ce que l'on utilise, comme catalyseurs, des phosphates de Al, B, Fe, Zr, Sr. Ce ou des mélanges de ceux-ci.

6. Procédé suivant les revendications 1 à 5, caractérisé en ce que l'on effectue l'isomérisation en phase gazeuse.